# EUROPEAN PATENT SPECIFICATION

(11) **EP 1 749 090 B1**
(45) Date of publication and mention of the grant of the patent: **17.05.2017**
(21) Application number: 05724065.7
(22) Date of filing: 01.03.2005
(51) Int. Cl.: A61K 35/14, C12N 5/0786

(54) **CELL THERAPY FORMULATION METHOD AND COMPOSITION**
ZELLTHERAPIEFORMULIERUNGSVERFAHREN UND ZUSAMMENSETZUNG
FORMULATION, PROCEDE ET COMPOSITION POUR THERAPIE CELLULAIRE

(30) Priority: 01.03.2004 US 549032 P
(43) Date of publication of application: 07.02.2007
(60) Divisional application: 17162282.2
(73) Proprietor: Immunovative Therapies, Ltd., 20179 Misgav (IL)
(72) Inventor: HAR-NOY, Michael, Modi'in, Jerusalem (IL)
(74) Representative: Lock, Graham James
(86) International application number: PCT/US2005/006446
(87) International publication number: WO 2005/084276

(56) References cited:
- WO-A-94/12196
- WO-A-03/038062
- WO-A-2005/001074
- WO-A-2005/081982
- WO-A1-94/12196
- US-A1- 2002 115 214
- US-B1- 6 534 055
- THOMAS A K ET AL: "A cell-based artificial antigen-presenting cell coated with anti-CD3 and CD28 antibodies enable rapid expansion and long-term growth of CD4 T lymphocytes" CLINICAL IMMUNOLOGY, ACADEMIC PRESS, US, vol. 105, no. 3, 3 December 2002 (2002-12-03), pages 259-272, XP002971111 ISSN: 1521-6616 -& WO 03/057171 A (TRUSTEES OF THE UNIVERSITY OF [US]; MAUS MARCELA [US]; THOMAS ANNA [DE) 17 July 2003 (2003-07-17)
- LUM L G ET AL: "Immune modulation in cancer patients after adoptive transfer of anti-CD3/anti-CD28-costimulated T cells-phase I clinical trial." JOURNAL OF IMMUNOTHERAPY (HAGERSTOWN, MD. : 1997) 2001 SEP-OCT, vol. 24, no. 5, September 2001 (2001-09), pages 408-419, XP002957619 ISSN: 1524-9557
- MA J ET AL: "USE OF ENCAPSULATED SINGLE CHAIN ANTIBODIES FOR INDUCTION OF ANTI-IDIOTYPIC HUMORAL AND CELLULAR IMMUNE RESPONSES" JOURNAL OF PHARMACEUTICAL SCIENCES, AMERICAN PHARMACEUTICAL ASSOCIATION. WASHINGTON, US, vol. 87, no. 11, November 1999 (1999-11), pages 1375-1378, XP000877492 ISSN: 0022-3549
- SINHA V R ET AL: "Biodegradable microspheres for protein delivery" JOURNAL OF CONTROLLED RELEASE, ELSEVIER, AMSTERDAM, NL, vol. 90, no. 3, 31 July 2003 (2003-07-31), pages 261-280, XP004440534 ISSN: 0168-3659

## Description

### FIELD OF INVENTION

This invention relates to methods for formulating ex-vivo prepared T-cells for infusion and a composition comprising a treatment effective amount of T-cells formulated for infusion by the methods.

### BACKGROUND OF THE INVENTION

Cell therapy methods have been developed in order to enhance the host immune response to tumors, viruses and bacterial pathogens. Cell therapy methods often involve the ex-vivo activation and expansion of T-cells. Examples of these type of treatments include the use of tumor infiltrating lymphocyte (TIL) cells (see U.S. Patent No. 5,126,132 issued to Rosenberg), cytotoxic T-cells (see U.S. Patent No. 6,255,073 issued to Cai, et al.; and U.S. Patent No. 5,846,827 issued to Celis, et al.), expanded tumor draining lymph node cells (see U.S. Patent No. 6,251,385 issued to Terman), and various other lymphocyte preparations (see U.S. Patent No. 6,194,207 issued to Bell, et al.; U.S. Patent No. 5,443,983 issued to Ochoa, et al.; U.S. Patent No 6,040,177 issued to Riddell, et al.; U.S. Patent No. 5,766,920 issued to Babbitt, et al.).

Lum L.G. et al: "Immune Modulation in Cancer Patients After Adoptive Transfer of Anti-CD3/Anti-CD28-Costimulated T Cells-Phase I Clinical Trial", Journal of Immunotherapy, 24(5) : 408-419, 2001-09, discloses peripheral blood mononuclear cells (PBMCs) activated in cell culture with anti-CD3/anti-CD28 coated beads. When needed for infusion, the activated cells, with the beads removed, are washed and suspended in an infusion medium.

For maximum effectiveness of T-cells in cell therapy protocols, the ex vivo activated T-cell population should be in a state of maximal activation upon infusion. Efforts for developing improved methods for producing more effective T-cells for use in cell therapy protocols have focused on the ex-vivo activation methods. However, ex-vivo activated cells need to be harvested and administered to patients to have a therapeutic effect. The harvesting of the T-cells removes them from the activating stimuli available in the ex-vivo cultures. Therefore, the longer the time from harvest to infusion, the lower the quality of the T-cells.

There is a need to develop T-cell formulations for infusion that maintain the cells in a state that can maximally orchestrate an immune response to cancer, infectious diseases and other disease states at both the time of infusion and while circulating in the blood. Efforts to maintain the activation state of T-cells at the time of infusion have most commonly involved the formulation of the T-cells with exogenous IL-2. Systemic IL-2 administration to patients has also been used to maintain the activation state of T-cells post-infusion. However, exogenous IL-2 administration is extremely toxic to patients and has not resulted in significant benefit to patients undergoing T-cell infusions.

### SUMMARY OF THE INVENTION

This invention includes ex-vivo prepared T-cells which are harvested from cell culture conditions and formulated in media suitable for infusion. The method of formulation involves: (1) labeling the cells with one or more agents which have reactivity for T-cell surface moieties capable of delivering activation signals upon cross-linking; (2) mixing the labeled cells with biodegradable nanospheres or microspheres coated with a material capable of cross-linking the agents attached to the T-cell surface moieties; (3) suspending the mixture of labeled T-cells and coated biodegradable cross-linking spheres in a medium suitable for infusion; (4) packaging the mixture in a suitable container, such as a syringe or an IV infusion bag; and (5) parenteral administration of said mixture to a patient.

Alternatively, the invention can be accomplished with a formulation method which involves: (1) mixing a population of T-cells with biodegradable nanospheres or microspheres coated with a first material and one or more second materials, whereas the first material binds the second materials and the second materials have reactivity for surface moieties on the T-cells, and whereas the interaction of the second materials with the T-cells causes the activation of the T-cells; (2) suspending the mixture of T-cells and the biodegradable spheres in a medium suitable for infusion; and (3) packaging the mixture in a suitable container, such as a syringe or an IV infusion bag; and (4) parenteral administration of the mixture to a patient.

In practice of the present invention, the T-cells intended for infusion are first harvested from the ex-vivo cell culture environment. The harvested cells are then either immediately formulated for infusion, cryopreserved for later formulation, or shipped to the patient location for formulation.

Upon formulation by the method of the present invention, all the T-cells for infusion will be maximally activated in a non-toxic delivery vehicle.

For the purposes of the present invention, all references to T-cells includes a population of cells with at least a portion of the cells containing T-cells. T-cells are cells which express TCR, including α/β and γ/δ TCRs. T-cells include all cells which express CD3, including T-cell subsets which also express CD4 and CD8. T-cells include both naive and memory cells and effector cells such as CTL. T-cells also include regulatory cells such as Th1, Tc1, Th2, Tc2, Th3, Treg, and Tr1 cells. T-cells also include NKT-cells and similar unique classes of the T-cell lineage.

### REFERRED EMBODIMENTS

The biodegradable spheres of the present invention are preferably manufactured from aliphatic polyesters, such as poly(lactic acid) (PLA), poly(glycolic acid) (PGA), copolymers of PLA and PGA (PLGA) or poly(carprolactone) (PCL), and polyanhydrides. The spheres are produced into small particle sizes of 0.1 to 500 microns, preferably less than 10 microns and most preferably less than 1 micron. Microspheres of this size range are capable of direct injection into the body by conventional methods. It is preferred that the coated spheres be designed to degrade in physiological fluids within 7 days, more preferably within 3 days.

The preferred first material for coating on the biodegradable spheres is polyclonal goat (or sheep) anti-mouse polyclonal antibodies. By way of example, this preferred first material can be used to cross-link mouse-derived monoclonal antibodies, or fragments or genetically engineered derivatives thereof, that have specificity for T-cell surface moieties. Thus, for example, the mixing of goat anti-mouse coated microspheres (or nanospheres) with human T-cells labeled with mouse anti-human CD3 and mouse anti-human CD28 mAbs will cause the cross-linking of the mouse mAbs on the human T-cells through the binding of the goat anti-mouse polyclonal antibody with the mouse mAbs. The cross-linking of the mAbs causes the activation of the T-cells. Alternatively, the anti-human CD3 and anti-CD28 can also be first bound, preferably in a 50/50 ratio, on the goat (or sheep) polyclonal antibody coated spheres and mixed with the T-cells. It will be obvious to those skilled in the art that many combinations of first materials and second materials can be used to accomplish the objective of cross-linking second agents attached to T-cell surface moieties in order to initiate signal transduction and activation of T-cells.

The mixture of T-cells with cross-linked surface moieties is suspended in infusion medium (e.g., isotonic solutions such as normal saline, 5% dextrose, Plasma-Lyte (Baxter) or Normasol (Abbott). In some embodiments, the infusion medium is supplemented with 0.5%-10% human serum albumen (HSA).

The mixture is preferably adjusted to a final T-cell concentration of between 1x 10⁷ to 1 x 10⁸ cells per ml of infusion media. In a preferred embodiment, 10⁹ T-cells are formulated in 100 ml of infusion media. The formulation is then packaged in one or more containers, such as syringes, plastic pouches, or plastic bottles.

According to one aspect, the invention provides a composition comprising a treatment effective amount of cells, of which a portion are T-cells, wherein the T-cells have been harvested from cell culture and are thereby removed from activating stimuli in the culture and the harvested T-cells are formulated for infusion by a method comprising (1) contacting the T-cells with one or more agents that ligate a cell surface moiety on at least a portion of the T-cells, (2) cross-linking the one or more agents with biodegradable nanospheres or microspheres coated with a material capable of cross-linking the one or more agents, (3) suspending the T-cells, the one or more agents and the coated biodegradable nanospheres or microspheres in an infusion medium, and (4) packaging in a suitable container, wherein the T-cells are in an activated state in the medium.

In one embodiment, the medium is suitable for parenteral infusion.

In another embodiment, the container is collapsible.

In a further embodiment, the container has a syringe portion.

According to another aspect, the invention provides a method for preparing a treatment effective amount of cells, of which a portion are T-cells, wherein the T-cells have been harvested from cell culture and are thereby removed from activating stimuli in the culture, the method comprising formulating the harvested T-cells for infusion by a method comprising: (1) labeling the harvested T-cells with one or more agents which have reactivity for T-cell surface moieties capable of delivering activation signals upon cross-linking; (2) mixing the labeled cells with biodegradable nanospheres or microspheres coated with a material capable of cross-linking the agents attached to the T-cell surface moieties; (3) suspending the mixture of labeled T-cells and coated biodegradable cross-linking spheres in an infusion medium, whereby the T-cells are in an activated state; and (4) packaging the mixture in a suitable container.

According to a further aspect, the invention provides a method for preparing a treatment effective amount of cells, of which a portion are T-cells, wherein the T-cells have been harvested from cell culture and are thereby removed from activating stimuli in the culture, the method comprising formulating the harvested T-cells for infusion by a method comprising: (1) mixing a population of the harvested T-cells with biodegradable nanospheres or microspheres coated with a first material and one or more second materials; wherein the first material cross-links the second materials, and the second materials have reactivity for surface moieties on the T-cells, and wherein the interaction of the second materials with the T-cells causes the activation of the T- cells; (2) suspending the mixture of T-cells and the biodegradable spheres in an infusion medium, whereby the T-cells are in an activated state; and (3) packaging the mixture in a container. Preferably, the package mixture of the T-cell concentration is at least I x 10⁷ cells per ml of infusion media.

According to the methods of the invention, the container is preferably a syringe or an iv infusion bag. More preferably, the container is collapsible. The collapsible container preferably includes opposing walls of flexible material and an exit tube.

## Claims

1. A composition comprising a treatment effective amount of cells, of which a portion are T-cells, wherein the T-cells have been harvested from cell culture and are thereby removed from activating stimuli in the culture and the harvested T-cells are formulated for infusion by a method comprising (1) contacting the T-cells with one or more agents that ligate a cell surface moiety on at least a portion of the T-cells, (2) cross-linking the one or more agents with biodegradable nanospheres or microspheres coated with a material capable of cross-linking the one or more agents, (3) suspending the T-cells, the one or more agents and the coated biodegradable nanospheres or microspheres in an infusion medium, and (4) packaging in a suitable container, wherein the T-cells are in an activated state in the medium.

2. The composition of claim 1, wherein the medium is suitable for parenteral infusion.

3. The composition of claim 1 or claim 2, whereby the container is collapsible.

4. The composition of any one of claims 1 to 3, whereby the container has a syringe portion.

5. A method for preparing a treatment effective amount of cells, of which a portion are T-cells, wherein the T-cells have been harvested from cell culture and are thereby removed from activating stimuli in the culture, the method comprising formulating the harvested T-cells for infusion by a method comprising: (1) labeling the harvested T-cells with one or more agents which have reactivity for T-cell surface moieties capable of delivering activation signals upon cross-linking; (2) mixing the labeled cells with biodegradable nanospheres or microspheres coated with a material capable of cross-linking the agents attached to the T-cell surface moieties; (3) suspending the mixture of labeled T-cells and coated biodegradable cross-linking spheres in an infusion medium, whereby the T-cells are in an activated state; and (4) packaging the mixture in a suitable container.

6. The method of claim 5 wherein the container is a syringe or an IV infusion bag.

7. The method of claim 5 wherein the container is collapsible.

8. The method of claim 7 wherein the container includes opposing walls of flexible material and an exit tube.

9. A method for preparing a treatment effective amount of cells, of which a portion are T-cells, wherein the T-cells have been harvested from cell culture and are thereby removed from activating stimuli in the culture, the method comprising formulating the harvested T-cells for infusion by a method comprising: (1) mixing a population of the harvested T-cells with biodegradable nanospheres or microspheres coated with a first material and one or more second materials; wherein the first material cross-links the second materials, and the second materials have reactivity for surface moieties on the T-cells, and wherein the interaction of the second materials with the T-cells causes the activation of the T- cells; (2) suspending the mixture of T-cells and the biodegradable spheres in an infusion medium, whereby the T-cells are in an activated state; and (3) packaging the mixture in a container.

10. The method of claim 9 wherein the container is a syringe or an IV infusion bag.

11. The method of claim 9 wherein the container is collapsible.

12. The method of claim 11 wherein the container includes opposing walls of flexible material and an exit tube.

13. The method of claim 9 wherein the package mixture of the T-cell concentration is at least I x 10⁷ cells per ml of infusion media.

## Patentansprüche

1. Zusammensetzung, welche eine Behandlungs-wirksame Menge von Zellen umfasst, von denen ein Teil T-Zellen sind, worin die T-Zellen aus einer Zellkultur geerntet wurden und dadurch von aktivierenden Stimuli in der Kultur befreit wurden, und worin die geernteten T-Zellen zur Infusion mittels eines Verfahrens formuliert werden, welches umfasst
(1) Inkontaktbringen der T-Zellen mit ein oder mehreren Mitteln, die eine Zelloberflächengruppe auf mindestens einem Bereich der T-Zellen ligieren,
(2) Vernetzen der ein oder mehreren Mittel mit bioabbaubaren Nanokügelchen oder Mikrokügelchen, die mit einem Material beschichtet sind, mit dem die ein oder mehreren Mittel vernetzt werden können,
(3) Suspendieren der T-Zellen, der ein oder mehreren Mittel und der beschichteten bioabbaubaren Nanokügelchen oder Mikrokügelchen in einem Infusionsmedium, und
(4) Verpacken in einem geeigneten Behälter,
wobei die T-Zellen in dem Medium in einem aktivierten Zustand sind.

2. Zusammensetzung nach Anspruch 1, worin das Medium zur parenteralen Infusion geeignet ist.

3. Zusammensetzung nach Anspruch 1 oder Anspruch 2, worin der Behälter zusammenklappbar ist.

4. Zusammensetzung nach einem der Ansprüche 1 bis 3, worin der Behälter einen Spritzen-Bereich aufweist.

5. Verfahren zur Herstellung einer Behandlungs-wirksamen Menge von Zellen, von denen ein Teil T-Zellen sind, worin die T-Zellen aus einer Zellkultur geerntet wurden und dadurch von aktivierenden Stimuli in der Kultur befreit wurden, worin das Verfahren umfasst, Formulieren der geernteten T-Zellen zur Infusion mittels eines Verfahrens, welches umfasst:
(1) Markieren der geernteten T-Zellen mit ein oder mehreren Mitteln, die reaktiv sind für T-Zell-Oberflächengruppen, die bei Vernetzung Aktivierungssignale liefern können;
(2) Mischen der markierten Zellen mit bioabbaubaren Nanokügelchen oder Mikrokügelchen, die mit einem Material beschichtet sind, mit dem die an die T-Zell-Oberflächengruppen gebundenen Mittel vernetzt werden können;
(3) Suspendieren des Gemisches markierter T-Zellen und beschichteter bioabbaubarer Vernetzungs-Kügelchen in einem Infusions-Medium, wobei die T-Zellen in einem aktivierten Zustand vorliegen; und
(4) Verpacken des Gemisches in einen geeigneten Behälter.

6. Verfahren nach Anspruch 5, wobei der Behälter eine Spritze oder ein IV Infusionsbeutel ist.

7. Verfahren nach Anspruch 5, wobei der Behälter zusammenfaltbar ist.

8. Verfahren nach Anspruch 7, wobei der Behälter gegenüberliegende Wände aus flexiblem Material und eine Ausgangsleitung aufweist.

9. Verfahren zur Herstellung einer Behandlungs-wirksamen Menge von Zellen, von denen ein Teil T-Zellen sind, worin die T-Zellen aus einer Zellkultur geerntet wurden und dadurch von aktivierenden Stimuli in der Kultur befreit wurden, worin das Verfahren umfasst, Formulieren der geernteten T-Zellen zur Infusion mittels eines Verfahrens, welches umfasst :
(1) Mischen einer Population geernteter T-Zellen mit bioabbaubaren Nanokügelchen oder Mikrokügelchen, die mit einem ersten Material und ein oder mehreren zweiten Materialien beschichtet sind; worin das erste Material die zweiten Materialien vernetzt, und worin die zweiten Materialien eine Reaktivität für Oberflächengruppen auf den T-Zellen aufweisen, und worin die Interaktion der zweiten Materialien mit den T-Zellen eine Aktivierung der T-Zellen bewirkt;
(2) Suspendieren des Gemisches von T-Zellen und der bioabbaubaren Kügelchen in einem Infusionsmedium, wobei die T-Zellen in einem aktivierten Zustand vorliegen; und
(3) Verpacken des Gemisches in einem Behälter.

10. Verfahren nach Anspruch 9, wobei der Behälter eine Spritze oder ein IV Infusionsbeutel ist.

11. Verfahren nach Anspruch 9, wobei der Behälter zusammenklappbar ist.

12. Verfahren nach Anspruch 11. wobei der Behälter gegenüberliegende Wände aus flexiblem Material und eine Auslassleitung aufweist.

13. Verfahren nach Anspruch 9 wobei das Packungs-Gemisch der T-Zell-Konzentration mindestens 1 x 10⁷ Zellen pro ml Infusionsmedium beträgt.

## Revendications

1. Composition comprenant une quantité thérapeutique efficace de cellules, dont une partie consiste en des lymphocytes T, dans laquelle les lymphocytes T ont été récoltés à partir d'une culture cellulaire et sont de cette façon dégagés des stimuli d'activation dans la culture et les lymphocytes T récoltés sont formulés pour une perfusion par un procédé comprenant (1) la mise en contact des lymphocytes T avec un ou plusieurs agents qui ligaturent une fraction de la surface cellulaire sur au moins une partie des lymphocytes T, (2) la réticulation des un ou plusieurs agents avec des nanosphères ou des microsphères biodégradables enrobées d'un matériau capable de réticuler les un ou plusieurs agents, (3) la mise en suspension des lymphocytes T, des un ou plusieurs agents et des nanosphères ou des microsphères biodégradables enrobées dans un milieu de perfusion, et (4) le conditionnement dans un récipient approprié, dans lequel les lymphocytes T sont dans un état activé dans le milieu.

2. Composition selon la revendication 1, dans laquelle le milieu est approprié pour une perfusion parentérale.

3. Composition selon la revendication 1 ou la revendication 2, auquel cas le récipient est compressible.

4. Composition selon l'une quelconque des revendications 1 à 3, auquel cas le récipient comporte une partie de seringue.

5. Procédé de préparation d'une quantité thérapeutique efficace de cellules, dont une partie consiste en des lymphocytes T, dans lequel les lymphocytes T ont été récoltés à partir d'une culture cellulaire et sont de cette façon dégagés des stimuli d'activation dans la culture, le procédé comprenant la formulation des lymphocytes T récoltés pour une perfusion par un procédé comprenant : (1) le marquage des lymphocytes T récoltés avec un ou plusieurs agents qui présentent une réactivité pour des fractions de la surface des lymphocytes T capables de délivrer des signaux d'activation lors de la réticulation ; (2) le mélange des cellules marquées avec des nanosphères ou des microsphères biodégradables enrobées d'un matériau capable de réticuler les agents fixés aux fractions de la surface des lymphocytes T ; (3) la mise en suspension du mélange des lymphocytes T marqués et des sphères biodégradables enrobées de réticulation dans un milieu de perfusion, auquel cas les lymphocytes T sont dans un état activé ; et (4) le conditionnement du mélange dans un récipient approprié.

6. Procédé selon la revendication 5, dans lequel le récipient est une seringue ou une poche de perfusion IV.

7. Procédé selon la revendication 5, dans lequel le récipient est compressible.

8. Procédé selon la revendication 7, dans lequel le récipient comprend des parois opposées de matériau flexible et un tuyau de sortie.

9. Procédé de préparation d'une quantité thérapeutique efficace de cellules, dont une partie consiste en des lymphocytes T, dans lequel les lymphocytes T ont été récoltés à partir d'une culture cellulaire et sont de cette façon dégagés des stimuli d'activation dans la culture, le procédé comprenant la formulation des lymphocytes T récoltés pour une perfusion par un procédé comprenant : (1) le mélange d'une population des lymphocytes T récoltés avec des nanosphères ou des microsphères biodégradables enrobées d'un premier matériau et d'un ou de plusieurs seconds matériaux ; dans lequel le premier matériau réticule les seconds matériaux, et les seconds matériaux présentent une réactivité pour des fractions de surface sur les lymphocytes T, et dans lequel l'interaction des seconds matériaux avec les lymphocytes T provoque l'activation des lymphocytes T ; (2) la mise en suspension du mélange des lymphocytes T et des sphères biodégradables dans un milieu de perfusion, auquel cas les lymphocytes T sont dans un état activé ; et (3) le conditionnement du mélange dans un récipient.

10. Procédé selon la revendication 9, dans lequel le récipient est une seringue ou une poche de perfusion IV.

11. Procédé selon la revendication 9, dans lequel le récipient est compressible.

12. Procédé selon la revendication 11, dans lequel le récipient comprend des parois opposées de matériau flexible et un tuyau de sortie.

13. Procédé selon la revendication 9, dans lequel dans le mélange du conditionnement, la concentration des lymphocytes T est d'au moins 1 x 10⁷ cellules par ml de milieu de perfusion.
